# EUROPÄISCHE PATENTSCHRIFT

(11) **EP 2 570 164 B1**
(45) Veröffentlichungstag und Bekanntmachung des Hinweises auf die Patenterteilung: **23.10.2013**
(21) Anmeldenummer: 11007558.7
(22) Anmeldetag: 16.09.2011
(51) Int. Cl.: B01D 19/00, B01D 53/14, B01D 53/24

(54) **Verfahren und Vorrichtung zur absorptiven Entfernung von Kohlendioxid aus Biogas**
Device and method for removing carbon dioxide from biogas by means of absorption
Procédé et dispositif d'elimination de dioxyde de carbone contenu dans du biogaz par absorption

(43) Veröffentlichungstag der Anmeldung: 20.03.2013
(73) Patentinhaber: MT-Biomethan GmbH, 27404 Zeven (DE)
(72) Erfinder: Engelke, Stephan, 21220 Maschen (DE); Jordan, Uwe, 27726 Worpswede (DE)
(74) Vertreter: Tragsdorf, Bodo

(56) Entgegenhaltungen:
- WO-A1-2008/000753
- WO-A1-2008/034473
- DE-A1-102009 013 883
- DE-B3-102005 051 952
- GB-A- 2 035 150
- US-A- 2 811 219

## Beschreibung

Die Erfindung bezieht sich auf ein Verfahren zur absorptiven Entfernung von Kohlendioxid aus Biogas unter Verwendung einer Waschflüssigkeit, in der Kohlendioxid chemisch gebunden wird, und eine zur Durchführung des Verfahrens geeignete Vorrichtung.

Biogas ist ein Gas, das aus nachwachsenden Rohstoffen oder biologischen Abfällen gewonnen wird. Die Hauptbestandteile von Biogas sind CO₂ und Methan. Soll Biogas in ein Erdgasnetz eingespeist werden, so müssen aus diesem CO₂ und andere unerwünschte Nebenprodukte entfernt werden.

Zur Entfernung von CO₂ aus Rohbiogas haben absorptive Trennverfahren wirtschaftliche Bedeutung erlangt, insbesondere solche, die mittels Waschlösungen bzw. Waschflüssigkeiten arbeiten, in denen die Gaskomponente CO₂ chemisch gebunden wird. Der Vorteil dieser Verfahren ist, dass durch Chemisorption eine vergleichsweise hohe Beladung der Waschflüssigkeit erreicht werden kann. Auch bei der Chemisorption werden kleinere Gasmengen noch physikalisch gebunden. Aufgrund der hohen Kosten für die Waschflüssigkeit ist es erforderlich, die beladene Waschflüssigkeit zu regenerieren. Der Absorptionsstufe wird hierzu in der Regel eine Desorptionsstufe nachgeschaltet.

Ein Verfahren zur absorptiven Entfernung von Kohlendioxid aus Biogas unter Verwendung einer aminhaltigen Waschflüssigkeit, in der Kohlendioxid chemisch gebunden wird, ist beispielsweise aus der DE 10 2009 056 661 A1 bekannt. Zur Absorption wird die Waschlösung im Gegenstrom mit dem zugeführten Biogas durch eine Absorbereinheit geleitet und die abgezogene beladene Waschlösung anschließend durch Desorption regeneriert.

Während der Desorption anfallendes Abgas enthält noch Restmengen an Methan, die als sogenannter Methanschlupf bezeichnet werden.

Ein Methanschlupf tritt bei allen bekannten Biogas-Trennverfahren auf, der je nach Trennverfahren bis zu 10 Vol.-% betragen kann.

Die im Abgas einer Biogasanlage emittierbare Methangasmenge ist gesetzlich begrenzt. Um diese Anförderungen zu erfüllen, ist es in der Praxis üblich, das Abgas thermisch oder katalytisch zu oxidieren. Gegebenenfalls müssen noch brennbare Gase zugesetzt werden. Die vorgenannte oxidative Behandlung des Abgases ist mit zusätzlichem Aufwand verbunden und unwirtschaftlich.

Die DE 10 2005 051 952 B3 und WO 2008/034473 A1 offenbaren ein Verfahren und eine Vorrichtung zur absorptiven Entfernung von Kohlendioxid aus Biogas mit den Merkmalen gemäß dem Oberbegriff des Anspruchs 1.

In der DE 10 2008 046 879 A1 wird vorgeschlagen, den Abgasstrom vor der Oxidation durch geschlossen ausgebildete Vodagebehälter und/oder Gärrestlager zu leiten, zur Inertisierung dort entstehender explosiver Gaskonzentrationen.

Der wesentliche Nachteil, der durch den Methanschlupf verursachte Verlust an Energie, kann jedoch mit dieser Lösung nicht beseitigt werden.

Aus der DE 10 2009 013 883 A1 ist eine Zentrifuge zur Trennung von Gasgemischen, beispielsweise auch Biogas, in ihre Komponenten nach ihrem jeweiligen Molekular- oder Atomgewicht bekannt.

Der Erfindung liegt die Aufgabe zugrunde, ein Verfahren zur absorptiven Entfernung von Kohlendioxid aus Biogas unter Verwendung einer Waschflüssigkeit, in der Kohlendioxid chemisch gebunden wird, zu schaffen, bei dem der Verlust an Methan verringert wird. Ferner soll eine zur Durchführung des Verfahrens geeignete Vorrichtung geschaffen werden.

Erfindungsgemäß wird die Aufgabe durch die im Anspruch 1 angegebenen Verfahrensmerkmale gelöst. Vorteilhafte Weiterbildungen der Verfahrensweise sind Gegenstand der Ansprüche 2 bis 10. Eine zur Durchführung des Verfahrens geeignete Vorrichtung ist Gegenstand von Anspruch 11. Die abhängigen Ansprüche 12 bis 15 beziehen sich auf vorteilhafte Ausgestaltungen der Vorrichtung.

Die nach der Absorption anfallende beladene Waschlösung wird bis auf eine Temperatur erwärmt, bei der eine Freisetzung von CO₂ beginnt. Diese Temperatur ist abhängig von der Zusammensetzung der Waschflüssigkeit und der Beladungsdichte der Waschflüssigkeit. Überraschenderweise zeigte sich in Versuchen, dass bei einer exakten Einhaltung der Temperatur, bei der die Freisetzung von CO₂ beginnt, nur eine effektive Abtrennung von Methan aus der beladenen Waschlösung gelingt. In den ersten 1 bis 2% an freigesetztem CO₂ ist nahezu die gesamte Restmenge an Methan (Methanschlupf) enthalten. Anschließend wird die beladene Waschlösung mindestens einem Zentrifugalabscheider zur Abtrennung der Gasphase von der flüssigen Phase zugeführt, wobei über die Gasphase Methan und gelöste Anteile an CO₂ entweichen. Die abgetrennte Gasphase wird in die Absorbereinheit geleitet und durchströmt diese zusammen mit dem zugeführten Rohbiogas.

Die abgetrennte flüssige Phase wird weiter auf die für die Desorption erforderliche Temperatur erwärmt und der Desorptionseinheit zur Regenerierung zugeführt. Vorzugsweise erfolgt die Erwärmung der beladenen Waschflüssigkeit auf Trenntemperatur in einem ersten Wärmetauscher mittels im Gegenstrom zugeführter regenerierter Waschflüssigkeit als Wärmeträger. Dies ist energetisch am wirtschaftlichsten.

Die auf Trenntemperatur erwärmte beladene Waschflüssigkeit wird unmittelbar nach dem Austritt aus dem Wärmetauscher tangential in den Zentrifugalabscheider eingeleitet.

Die genaue Trenntemperatur wird in Abhängigkeit von der Beladung der Waschflüssigkeit vorab in Vorversuchen ermittelt.

Die aus dem Zentrifugalabscheider abgeführte flüssige Phase, eine nahezu methanfreie CO₂ beladene Waschflüssigkeit, wird durch einen zweiten Wärmetauscher geleitet und in diesem bis auf die erforderliche Desorptionstemperatur erwärmt, mittels Im Gegenstrom zugeführter regenerierter Waschflüssigkeit als Wärmeträger. Abschließend wird die beladene Waschlösung in der Desorptionseinheit regeneriert und für einen erneuten Einsatz zum Kopf der Absorptionseinheit geleitet.

Das aus der Waschlösung ausgetriebene CO₂ kann zum Beispiel zur Begasung von Gewächshäusern oder zur Synthese organischer oder anorganischer kohlenstoffhaltiger Verbindungen genutzt werden.

Vorzugsweise wird Im Zyklonabscheider der Druck der Gasphase geregelt, wobei durch die Druckregelung Temperaturschwankungen im Zentrifugalabscheider und Änderungen in der Beladung der zugeführten Waschflüssigkeit ausgeglichen werden.

Im stationären Betriebszustand wird der Druck im Zentrifugalabscheider so eingestellt, dass eine vorbestimmte Gasmenge abströmt und bei gewünschter Veränderung der abströmenden Gasmenge der Druck der Gasphase entsprechend verändert wird.

Im ersten Wärmetauscher wird die Temperatur der beladenen Waschlösung überwacht und in Abhängigkeit von der gemessenen Temperatur die Wärmezufuhr gesteuert. Diese Prozessstufe ist regelungstechnisch in die zentrale Steuerung der Biogasaufbereitungsanlage integriert.

Als Zentrifugalabscheider wird bevorzugt ein Zyklon eingesetzt. Ein derartiger Zyklon für eine Biogasaufbereitungsanlage mittlerer Baugröße mit einem Durchsatz an Waschflüssigkeit von ca. 10.000 bis 100.000 l/h besitzt ein Nutzvolumen von ca. 8 bis 140 Liter (Durchmesser von 100 bis 300 mm Höhe 1000 bis 2000 mm).

In Abhängigkeit von der Menge an anfallender beladener Waschlösung nach der Absorption können anstelle eines Zyklons auch mehrere, in der Regel kleinere, Zyklone eingesetzt werden, die entweder in Reihen- oder Parallelschaltung betrieben werden. Besonders gut geeignet ist eine Waschflüssigkeit, die chemische Substanzen enthält, die CO₂ in Form von Carbonat oder Hydrogencarbonat binden, wie primäre, sekundäre oder tertiäre Amine, Alkalisalze von Aminosäuren, Alkalicarbonatlösungen, einzeln oder als Gemische.

Eine zur Durchführung des Verfahrens geeignete Vorrichtung besteht aus einer Absorbereinheit mit mindestens einem Absorber und einer Desorbereinheit mit mindestens einem Desorber, wobei der Sumpf des Absorbers über eine beladene Waschflüssigkeit führende Leitung, in die mindestens ein Wärmetauscher eingebunden ist, mit dem Desorber verbunden ist. Der Desorber Ist über eine gereinigte Waschflüssigkeit führende Leitung mit dem Kopf des Absorbers verbunden.

Nach dem ersten Wärmetauscher ist in die die beladene Waschflüssigkeit führende Leitung mindestens ein Zentrifugalabscheider eingebunden. Die Zufuhrleitung mündet tangential in diesen. Durch die im Betriebszustand anliegende Zentrifugalkraft sammelt sich die abgetrennte Gasphase in der Rotationsachse. Die flüssige Phase wird an die Innenwandung des zentrifugalabscheiders gedrückt und fließt nach unten ab.

Am Kopf des Zentrifugalabscheiders ist eine Abgasleitung angeordnet, die unterhalb der Absorberschicht in den Absorber mündet. Die vom Zentrifugalabscheider abführende Flüssigkeitsleitung ist mit dem Kopf des Desorbers verbunden, wobei in die Flüssigkeitsleitung ein zweiter Wärmetauscher eingebunden ist.

Vorzugsweise ist im unteren Abschnitt des Zentrifugalabscheiders ein Prallblech zentrisch angeordnet, derart, dass zwischen der Wandung des Zentrifugalabscheiders und dem äußeren Rand des Prallbleches ein schmaler Ringkanal als Abströmöffnung für die Flüssigphase gebildet ist. Durch den schmalen Ringkanal entsteht im Betriebszustand ein wulstartiger Flüssigkeitsstau, durch den verhindert wird, dass eventuell Gas mit abströmen kann.

Im oberen Abschnitt des Zentrifugalabscheiders ist ein Tauch- bzw. Steigrohr angeordnet, das mit einem schwimmergesteuerten Entlüftungsventil verbunden ist. In bestimmten Anwendungsfällen kann es auch zweckmäßig sein, dass an das Tauch- bzw. Steigrohr noch eine Vakuumpumpe angeschlossen wird.

In der Abgasleitung des Zentrifugalabscheiders sind ein Durchflussmesser und ein Stellventil und in der Flüssigkeit abführenden Leitung des Zentrifugalabscheiders Ist ein Drucksensor angeordnet, die regelungstechnisch miteinander verbunden sind.

Mit der vorgeschlagenen Lösung lässt sich der Methanschlupf nahezu 100%ig beseitigen. Das freigesetzte Methan wird zusammen mit dem Hauptgasstrom an Methan einer energetischen Verwertung, z.B. Einspeisung in das Erdgasnetz, zugeführt. Dadurch wird die Wirtschaftlichkeit der Biogasanlage verbessert, da der Methanschlupf, der ansonsten ein Verlust ist, die Ausbeute an Methangas erhöht.

Ein weiterer Vorteil besteht darin, dass beim Einsatz von Waschlösungen nicht mehr darauf geachtet werden muss, wie hoch deren Methanschlupf ist. Dadurch können auch kostengünstigere Waschmittel zum Einsatz kommen.

Die Erfindung wird nachstehend an einem Ausführungsbeispiel erläutert, In der zugehörigen Zeichnung zeigen:
- Fig. 1: eine erfindungsgemäße Vorrichtung in vereinfachter schematischer Darstellung und
- Fig. 2: einen Zyklon als Einzelbauteil in vereinfachter Querschnittsdarstellung.

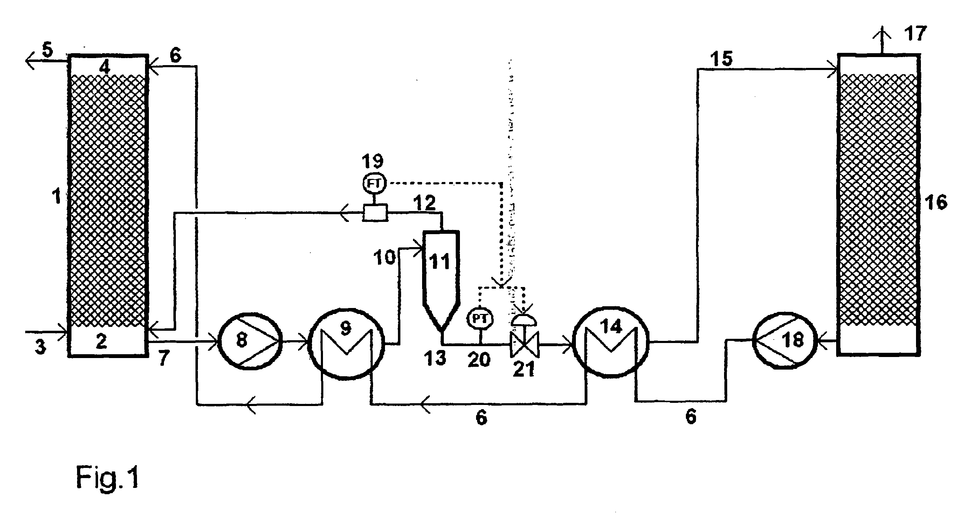

Die in Figur 1 gezeigte Vorrichtung besteht aus den Hauptbaugruppen Absorber 1, erster Wärmetauscher 9, Zyklon 11, zweiter Wärmetauscher 14 und Desorber 16.

Der Absorber 1 ist im unteren Abschnitt 2 (Sumpf) mit einer Leitung 3 für das zu reinigende Biogas (Rohgas) verbunden..Das gereinigte Biogas (Methan) wird im oberen Abschnitt 4 (am Kopf) über eine Leitung 5 abgeführt. Die über die Leitung 6 zugeführte Waschflüssigkeit wird im Absorber 1 fein verteilt. Das Rohbiogas wird im Gegenstrom zur Waschflüssigkeit durch die Absorptionskolonne 1 geleitet.

Bei einer Temperatur von ca. 40 °C nimmt die Waschflüssigkeit, z.B. eine Aminlösung, das im Biogas enthaltene Kohlendioxid auf. Das gereinigte Biogas (Methan) wird am Kopf der Absorptionskolonne 1 abgezogen. Im Sumpf 2 des Absorbers 1 wird über eine Leitung 7, in die eine erste Pumpe 8 eingebunden ist die beladene Waschflüssigkeit abgepumpt und einem ersten Wärmetauscher 9 zugeführt. Im Wärmetauscher 9 wird die beladene Waschflüssigkeit mittels regenerierter Waschflüssigkeit als Wärmeträger auf die erforderliche Trenntemperatur erhitzt. Ist diese erreicht, so beginnt in dem ersten Wärmetauscher 9 ein Freisetzen von CO₂. Das ist der Zeltpunkt, zu dem die beladene Waschflüssigkeit über die Leitung 10 unter definiertem Druck in den Zyklon 11 gepumpt wird. Überraschenderweise wurde festgestellt, dass in den ersten 1 bis 2% an freigesetztem CO₂ bereits nahezu bis zu 100% des Methanschlupfes enthalten sind. Demzufolge muss die Erwärmung der beladenen Waschflüssigkeit bei dieser Temperatur unterbrochen und diese dem Zyklon 11 zur Trennung der Gasphase von der Flüssigphase zugeführt werden. Die Zuführung in den Zyklon 11 erfolgt tangential im oberen Abschnitt. Auf den Aufbau des Zyklons wird nachfolgend noch näher eingegangen. Im Zyklon 11 erfolgt eine Trennung der zugeführten beladenen Waschflüssigkeit in eine methanreiche Gasphase und eine nahezu methanfreie Flüssigphase. Die Gasphase wird am Kopf des Zyklons 11 über eine Leitung 12 abgezogen und am unteren Abschnitt 2 in den Absorber 1 geleitet, wo sie zusammen mit dem zugeführten Rohbiogas aufsteigt. Die am kegelförmigen Auslass des Zyklons 11 anfallende beladene Waschflüssigkeit wird über eine Leitung 13 abgepumpt und einem zweiten Wärmetauscher 14 zur Erhitzung auf Desorptionstemperatur zugeführt. Im zweiten Wärmetauscher 14 wird die beladene Waschflüssigkeit mittels regenerierter Waschflüssigkeit als Wärmeträger auf die erforderliche Temperatur erhitzt und über die Leitung 15 am Kopf der Desorptionskolonne 16 in diese unter erhöhtem Druck eingeleitet und nachfolgend entspannt. Die Desorption kann auch durch eine mehrstufige Entspannung in an sich bekannter Weise erfolgen. Durch die Entspannung entweicht das in der Waschflüssigkeit gebundene CO₂. Das anfallende Gasgemisch (CO₂, Wasser und Schwefalverbindungen) wird als Brüden über eine am Kopf der Desorptionskolonne 16 angeordnete Leitung 17 abgezogen und kann als Wärmeträger genutzt und nachfolgend weiter aufbereitet werden.

Die am Sumpf der Desorptionskolonne 16 anfallende regenerierte Waschflüssigkeit mit einer Temperatur von ca. 120 bis 150 °C wird als Wärmeträger genutzt und über die Leitung 6, in die eine zweite Pumpe 18 eingebunden ist, durch den zweiten Wärmetauscher 14 und nachfolgend durch den ersten Wärmetauscher 9 gepumpt und dabei bis auf ca. 40 bis 50 °C abgekühlt und dem Absorber 1 zugeführt.

Die Trennung der beladenen Waschflüssigkeit in eine Gasphase und eine Flüssigphase kann mittels eines geregelten oder ungeregelten Zentrifugalabscheiders erfolgen. In der Figur 1 ist eine Ausführungsvariante mit einem geregelten Zyklon 11 gezeigt.

Bei einem ungeregelten Zentrifugalabscheider bzw. Zyklon kann eine Änderung des Betriebszustandes, wie zum Beispiel eine Änderung der absorbierten Menge CO₂ in der Absorbereinheit 1, lediglich über eine Veränderung der Temperaturen im Absorber 1 und Desorber 16 kompensiert werden. Dieser Maßnahme sind aber durch die vorgegebene Betriebsführung der Biogasaufbereitungsanlage enge Grenzen gesetzt.

Der im Zyklon 11 abgetrennte und über die Leitung 12 abgeführte Gasstrom kann durch eine Druckregelung gesteuert werden. Zur Druckregelung sind in die Abgasleitung 12 ein Durchflussmesser 19 und in die am Zyklonaustritt angeschlossene Leitung 13 ein Drucksensor 20 und ein Regelventil 21 eingebunden. Diese sind regelungstechnisch miteinander verbunden, was durch die gestrichelte Linie angedeutet ist.

Wird der Druck im Zyklon 11 erhöht, dann setzt das Ausgasen bei Eintritt in den Zyklon 11 bei einer höheren Temperatur ein. Über die Druckregelung im Zyklon 11 können Änderungen der Temperatur im Zyklon 11 kompensiert werden. Ein Regelbereich des Druckes von 2 bis 8 bar ermöglicht Temperaturschwankungen über eine Spanne von bis zu 20 K zu kompensieren.

Des Weiteren kann mit der Druckregelung auf Änderungen im Beladungszustand der Waschlösung reagiert werden. Wird der Druck Im Zyklon 11 erhöht, so kann aus einer höher beladenen Waschlösung im Zyklon 11 nahezu die gesamte Menge an Methan abgetrennt werden. Wird dagegen der Druck im Zyklon verringert, so kann eine geringer beladene Waschlösung im Zyklon 11 behandelt werden. Bei einem Regelbereich von 3 bis 6 bar können Beladungsschwankungen über eine Spanne von bis zu 20 g/l kompensiert werden.

Regelungstechnisch wird im Betriebszustand so verfahren, dass als Sollwert eine voreingestellte definierte Gasmenge aus dem Zyklon strömt. Soll die abströmende Gasmenge erhöht werden, so wird der Druck im Zyklon verringert. Zur Verringerung der Gasmenge wird der Druck im Zyklon erhöht.

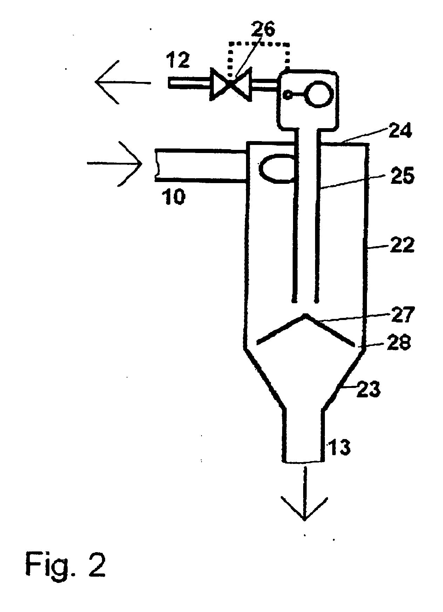

Der eingesetzte Zyklon 11 ist als Einzelbauteil in Figur 2 in vergrößerter Darstellung gezeigt.

Der Zyklon 11 besteht aus Edelstahl und hat einen Durchmesser von 200 mm und eine Bauhöhe von 1500 mm. Der zylinderförmige Abschnitt 22 hat eine Länge bzw. Höhe von 750 mm. Im unteren Abschnitt besitzt der Zyklon 11 einen kegelförmigen Auslauf 23 an den die Leitung 13 angeschlossen ist. Am oberen Enden des Zyklons 11 ist eine Abdeckung 24 angeordnet. Diese besitzt eine zentrale Öffnung, in die ein Tauchrohr 25 eingesetzt ist, das nach unten in den Zyklon 11 ragt und in der Rotationsachse des Zyklons 11 angeordnet ist. Im Tauchrohr 25 steigen die während des Trennvorganges gebildeten Gasblasen auf. Am nach oben überstehenden Ende des Tauchrohrs 25 ist ein schwimmergesteuertes Entlüftungsventil 26 angeordnet, das in Fig. 1 nicht mit gezeigt ist. Dieses bewirkt, dass nur gasförmige Stoffe den Zyklon 11 über die Leitung 12 verlassen können.

Im unteren Bereich des Zyklons 11 ist ein kegelförmiges Prallblech 27 angeordnet, dessen Spitze nach oben zeigt. Das Prallblech 27 erstreckt sich radial so weit nach außen, dass nur noch ein schmaler Ringkanal 28 gebildet wird. Dadurch wird gewährleistet, dass nur Flüssigkeit abströmen kann und keine Gasblasen.

### Beispiel 1

Als Waschlösung für die Absorption des zugeführten Biogases (Rohgases) wird eine handelsübliche wässrige Lösung von MDEA und Piperazin verwendet, mit Konzentrationen von 3,5 mol/l MDEA und 0,5 mol/l Piperazin. Die Waschlösung hat nach der Absorption eine Temperatur von 50 °C. Die Beladungen mit Kohlendioxid und Methan betragen 45 g/l bzw. 0,07 g/l (Methanschlupf).

Die beladene Waschlösung wird im ersten Wärmetauscher 9 bei einem Druck von 6 bar auf eine Temperatur von 112 °C erwärmt. Bei Erreichen dieser Temperatur wird die beladene Waschlösung tangential in den Zyklon 11 eingeleitet.

Unter diesen Bedingungen bildet sich kurz vor der Einleitung in den Zyklon 11 eine Gasphase mit folgender Zusammensetzung: CO₂: 72,5 Vol.-%; CH₄: 6,5 Vol.-%; Wasserdampf: 21 Vol.-%.

Die Beladung der flüssigen Phase mit Kohlendioxid beträgt 44,8 g/l. Die Beladung der flüssigen Phase mit Methan beträgt 0,0014 g/l.

Es sind 98% des Methans und nur 0,4% des Kohlendioxids in die Gasphase übergegangen. Im Zyklon 11 wird die die Gasphase von der flüssigen Phase abgetrennt und über die Leitung 12 in den Absorber 1 zum Biogasstrom geleitet.

### Beispiel 2

Die Zusammensetzung der verwendeten Waschlösung ist die gleiche wie in Beispiel 1.

Nach dem Austritt aus dem Absorber 1 beträgt die Beladung der Waschlösung mit Kohlendioxid 40 g/l. Die Waschlösung wird unter gleichen Bedingungen wie in Beispiel 1 im ersten Wärmetauscher auf 112 °C erwärmt. Die unter diesen Bedingungen entstehende Gasphase hat folgende Zusammensetzung: 20,4 Vol.-% Methan, 60,6 Vol.-% CO₂ und 21 Vol.-% Wasserdampf. In der flüssigen Phase sind noch 0,055 g/l Methan gelöst. Es sind also nur 22% des Methans in die Gasphase übergetreten.

Um einen möglichst geringen Methanschlupf zu erreichen, wird der Druck im Zyklon 11 auf 5,1 bar gesenkt. Die Temperatur von 112°C bleibt nahezu konstant. Durch den abgesenkten Druck gehen größere Mengen an Methan und Kohlendioxid in die Gasphase über. Diese besteht aus 14 Vol.-% Methan, 63,1 Vol.-% CO₂ und 22,8 Vol.-% Wasserdampf.

In der flüssigen Phase sind noch 0,008 g/l Methan gelöst. Es sind 88% des Methans in die Gasphase übergetreten. Die Gasphase wird im Zyklon von der Flüssigphase abtrennt und gelangt in den Absorber 1.

### Beispiel 3

Es wird analog wie in Beispiel 1 verfahren, lediglich mit dem Unterschied, dass die Temperatur der Waschlösung nach der Absorption 60 °C beträgt. Im ersten Wärmetauscher muss die beladene Waschlösung bis auf eine Temperatur von 117 °C erhitzt werden, um diese anschließend in den Zyklon 11 pumpen zu können. Bei dieser Temperatur entsteht eine Gasphase mit folgender Zusammensetzung: 0,2 Vol.-% Methan, 77 Vol.-% CO₂ und 22,7 Vol.-% Wasserdampf.

Die flüssige Phase enthält kein Methan aber 38,8 g/l CO₂ in gelöster Form.

Unter diesen Bedingungen würde bei einer Abtrennung der Gasphase von der Flüssigphase in einem zentrifugalabscheider viel zu viel CO₂ mit abgetrennt und im Kreislauf gefahren, was unwirtschaftlich wäre.

Dies wird ausgeschlossen, indem der Druck im Zyklon auf 7,5 bar erhöht wird. Dadurch geht weniger CO₂ in die Gasphase über.

Die unter diesen Bedingungen entstehende Gasphase enthält 10,8 Vol.-% Methan, 70,6 Vol.-% CO₂ und 18,6 Vol,-% Wasserdampf. In der flüssigen Phase verbleiben 0,0068 g/l Methan und 44,89 g/l CO₂. Es sind also über 90% des Methans in die Gasphase übergetreten. Die aus dem Zyklon 11 austretende methanreiche Gasphase gelangt in den Absorber 1.

Die Beispiele belegen, dass mit der vorgeschlagenen Verfahrensweise bei der Regenerierung einer beladenen Waschflüssigkeit der Methanschlupf um ca. 90 bis 98% verringert werden kann.

## Patentansprüche

1. Verfahren zur absorptiven Entfernung von Kohlendioxid aus Biogas unter Verwendung einer Waschflüssigkeit, in der Kohlendioxid chemisch gebunden wird, wobei die Waschlösung zur Absorption im Gegenstrom mit dem zugeführten Biogas durch eine Absorbereinheit (1) geleitet wird und die abgezogene beladene Waschflüssigkeit durch Desorption regeneriert wird, **dadurch gekennzeichnet, dass** die nach der Absorption anfallende beladene Waschflüssigkeit bis auf eine Temperatur erwärmt wird, bei der eine Freisetzung von CO₂ beginnt, und unmittelbar danach die beladene Waschflüssigkeit mindestens einem Zentrifugalabscheider (11) zur Abtrennung der Gasphase von der flüssigen Phase zugeführt wird, wobei über die Gasphase Methan und gelöste Anteile an CO₂ entweichen, und nach erfolgter Trennung die Gasphase in die Absorbereinheit (1) geleitet und die flüssige Phase weiter auf die für die Desorption erforderliche Temperatur erwärmt und der Desorptionseinheit (16) zur Regenerierung zugeführt wird.

2. Verfahren nach Anspruch 1, **dadurch gekennzeichnet, dass** die nach der Absorption abgezogene beladene Waschflüssigkeit durch einen ersten Wärmetauscher (9) geleitet wird und in diesem bis auf die erforderliche Trenntemperatur erwärmt wird, mittels im Gegenstrom zugeführter regenerierter Waschflüssigkeit als Wärmeträger, und anschließend tangential in den Zentrifugalabscheider (11) eingeleitet wird.

3. Verfahren nach einem der Ansprüche 1 oder 2, **dadurch gekennzeichnet, dass** die aus dem Zentrifugalabscheider (11) abgeführte flüssige Phase durch einen zweiten Wärmetauscher (14) geleitet wird und in diesem bis auf die erforderliche Desorptionstemperatur erwärmt wird, mittels im Gegenstrom zugeführter regenerierter Waschflüssigkeit als Wärmeträger, und anschließend in die Desorptionseinheit (16) gelangt.

4. Verfahren nach einem der Ansprüche 1 bis 3, **dadurch gekennzeichnet, dass** im Zentrifugalabscheider der Druck der Gasphase geregelt wird, wobei durch die Druckregelung Temperaturschwankungen im Zentrifugalabscheider (11) und Änderungen in der Beladung der zugeführten Waschflüssigkeit ausgeglichen werden.

5. Verfahren nach einem der Ansprüche 1 bis 4, **dadurch gekennzeichnet, dass** im stationären Betriebszustand der Druck im Zentrifugalabscheider (11) so eingestellt wird, dass eine vorbestimmte Gasmenge abströmt und bei gewünschter Veränderung der abströmenden Gasmenge der Druck verändert wird.

6. Verfahren nach einem der Ansprüche 2 bis 5, **dadurch gekennzeichnet, dass** im ersten Wärmetauscher (9) die Temperatur der beladenen Waschlösung überwacht und in Abhängigkeit von der gemessenen Temperatur die Wärmezufuhr gesteuert wird.

7. Verfahren nach einem der Ansprüche 1 bis 6, **dadurch gekennzeichnet, dass** als Zentrifugalabscheider ein Zyklon (11) eingesetzt wird.

8. Verfahren nach einem der Ansprüche 1 bis 6, **dadurch gekennzeichnet, dass** mehrere Zyklone eingesetzt werden, die entweder in Reihen- oder Parallelschaltung betrieben werden.

9. Verfahren nach einem der Ansprüche 1 bis 8, **dadurch gekennzeichnet, dass** eine Waschflüssigkeit eingesetzt wird, die chemische Substanzen enthält, die CO₂ in Form von Carbonat oder Hydrogencarbonat binden.

10. Verfahren nach Anspruch 9, **dadurch gekennzeichnet, dass** als chemische Substanzen primäre, sekundäre oder tertiäre Amine, Alkalisalze von Aminosäuren, Alkalicarbonatlösungen, einzeln oder als Gemische, eingesetzt werden.

11. Vorrichtung zur Durchführung des Verfahrens nach einem der Ansprüche 1 bis 10, bestehend aus einer Absorbereinheit mit mindestens einem Absorber (1) und einer Desorbereinheit mit mindestens einem Desorber (16), wobei der untere Abschnitt (2) des Absorbers (1) über eine beladene Waschflüssigkeit führende Leitung (7), in die mindestens ein Wärmetauscher eingebunden ist, mit dem Desorber (16) verbunden ist, und vom Desorber (16) eine gereinigte Waschflüssigkeit führende Leitung (6) zum Kopf des Absorbers (1) führt, **dadurch gekennzeichnet, dass** nach dem ersten Wärmetauscher (9) in die die beladene Waschflüssigkeit führende Leitung (7) mindestens ein Zentrifugalabscheider (11) eingebunden ist, wobei die Zufuhrleitung (10) In den Zentrifugalabscheider (11) tangential in diesen mündet, am Kopf des Zentrifugalabscheiders (11) eine Abgasleitung (12) angeordnet ist, die zum unteren Abschnitt (2) des Absorbers (1) führt, und die vom Zentrifugalabscheider (11) abführende Flüssigkeitsleitung (13) mit dem Kopf des Desorbers (16) verbunden ist, wobei in diese ein zweiter Wärmetauscher (14) eingebunden ist.

12. Vorrichtung nach Anspruch 11, **dadurch gekennzeichnet, dass** als Zentrifugalabscheider ein einzelner Zyklon (11) oder mehrere in Reihe oder parallel geschaltete Zyklone angeordnet sind.

13. Vorrichtung nach einem der Ansprüche 11 oder 12, **dadurch gekennzeichnet, dass** im unteren Abschnitt des Zentrifugalabscheiders (11) ein Prallblech (27) zentrisch angeordnet ist, derart, dass zwischen der Wandung des Zentrifugalabscheiders (11) und dem äußeren Rand des Prallbleches (27) ein schmaler Ringkanal (28) als Abströmöffnung für die Flüssigphase gebildet ist.

14. Vorrichtung nach einem der Ansprüche 11 bis 13, **dadurch gekennzeichnet, dass** im oberen Abschnitt des Zentrifugalabscheiders (11) ein Tauchrohr (25) angeordnet ist, das mit einem schwimmergesteuerten Entlüftungsventil (26) und gegebenenfalls mit einer Vakuumpumpe verbunden ist.

15. Vorrichtung nach einem der Ansprüche 11 bis 14, **dadurch gekennzeichnet, dass** in der Abgasleitung (12) des Zentrifugalabscheiders (11) ein Durchflussmesser (19) und in der Flüssigkeit abführenden Leitung (13) des Zentrifugalabscheiders (11) ein Drucksensor (20) und ein Stellventil (21) angeordnet sind, die regelungstechnisch miteinander verbunden sind.

## Claims

1. A method for the absorptive removal of carbon dioxide from biogas using a scrubbing liquid in which carbon dioxide is chemically bound, wherein the scrubbing solution, for absorption, is passed in counterflow with the fed biogas through an absorber unit (1) and the loaded scrubbing liquid that is withdrawn is regenerated by desorption, **characterized in that** heating the loaded scrubbing liquid occurring after the absorption to a temperature at which liberation of CO₂ begins, and immediately thereafter feeding the loaded scrubbing liquid to at least one centrifugal separator (11) for separating off the gas phase from the liquid phase, wherein methane and dissolved fractions of CO₂ escape via the gas phase, and after separation is complete, passing the gas phase into the absorber unit (1) and heating the liquid phase further to the temperature necessary for desorption, and feeding it to the desorption unit (16) for regeneration.

2. Method according to claim 1, **characterized in that** the loaded scrubbing liquid that is withdrawn is passed after the absorption through a first heat exchanger (9) and heated therein to the required separation temperature using regenerated scrubbing liquid fed in counterflow as heat carrier, and subsequently introduced tangentially into the centrifugal separator (11).

3. Method according to claim 1 or 2, **characterized in that** the liquid phase removed from the centrifugal separator (11) is passed through a second heat exchanger (14) and heated therein to the required desorption temperature using regenerated scrubbing liquid fed in counterflow as heat carrier, and subsequently passes into the desorption unit (16).

4. Method according to one of the claims 1 to 3, **characterized in that** the pressure of the gas phase in the centrifugal separator is controlled, wherein temperature fluctuations in the centrifugal separator (11) and changes in the loading of the scrubbing liquid that is fed can be compensated for by the pressure controller.

5. Method according to one of the claims 1 to 4, **characterized in that** in the steady operating state, the pressure in the centrifugal separator (11) is adjusted in such a manner that a predetermined amount of gas flows off and the pressure is altered in the event of a desired change in the amount of gas flowing off.

6. Method according to one of the claims 2 to 5, **characterized in that** in the first heat exchanger (9), the temperature of the loaded scrubbing solution is monitored and the heat supply is controlled in dependence on the measured temperature.

7. Method according to one of the claims 1 to 6, **characterized in that** as centrifugal separator, a cyclone (11) is used.

8. Method according to one of the claims 1 to 6, **characterized in that** a plurality of cyclones are used that are operated either in series or parallel connection.

9. Method according to one of the claims 1 to 8, **characterized in that** a scrubbing liquid is used that contains chemical substances that bind CO₂ in the form of carbonate or hydrogencarbonate.

10. Method according to claim 9, **characterized in that** as chemical substances, primary, secondary or tertiary amines, alkali metal salts of amino acids, alkali metal carbonate solutions, are used individually or as mixtures.

11. Device for carrying out the method in accordance with one of the claims 1 to 10 consisting of an absorber unit having at least one absorber (1) and a desorber unit having at least one desorber (16), wherein the bottom section (2) of the absorber (1) is connected via a line (7) bearing a loaded scrubbing liquid, into which line at least one heat exchanger is incorporated, is connected to the desorber (16), and from the desorber (16), a line (6) bearing purified scrubbing liquid leads to the top of the absorber (1), **characterized in that** downstream of the first heat exchanger (9) at least one centrifugal separator (11) is incorporated into the line (7) bearing the loaded scrubbing liquid, wherein the feed line (10) into the centrifugal separator (11) opens out tangentially therein, at the top of the centrifugal separator (11), an off-gas line (12) is arranged which leads to the bottom section (2) of the absorber (1), and the liquid line (13) leading away from the centrifugal separator (11) is connected to the top of the desorber (16), wherein a second heat exchanger (14) is incorporated therein.

12. Device according to claim 11, **characterized in that** as centrifugal separator, a single cyclone (11) or a plurality of series- or parallel-connected cyclones are arranged.

13. Device according to claim 11 or 12, **characterized in that** in the bottom section of the centrifugal separator (11), a baffle plate (27) is centrally arranged in such a manner that, between the wall of the centrifugal separator (11) and the outer rim of t he baffle plate (27), a narrow annular channel (28) is formed as outlet opening for the liquid phase.

14. Device according to one of the claims 11 to 13, **characterized in that** in the top section of the centrifugal separator (11), a submerged tube (25) is arranged that is connected to a float-controlled deaerating valve (26) and optionally to a vacuum pump.

15. Device according to one of the claims 11 to 14, **characterized in that** in the off-gas line (12) of the centrifugal separator (11), a flow meter (19) is arranged, and in the line (13) of the centrifugal separator (11) leading away the liquid, a pressure sensor (20) and a control valve (21) are arranged, which are interconnected via a control system.

## Revendications

1. Procédé pour l'élimination par absorption du dioxyde de carbone contenu dans du biogaz en utilisant un liquide lavant dans lequel le dioxyde de carbone est lié chimiquement, la solution lavante traversant une unité d'absorbants (1) en vue de l'absorption à contre-courant avec le biogaz amené et le liquide lavant soutiré et chargé étant régénéré par désorption, **caractérisé en ce que** le liquide lavant chargé, produit après l'absorption, est porté à une température à laquelle le CO₂ commence à se libérer et, directement après, le liquide lavant chargé est amené dans au moins un séparateur centrifuge (11) destiné à séparer la phase gazeuse de la phase liquide, du méthane et des particules de CO₂ détachées s'échappant grâce à la phase gazeuse et, au terme de la séparation, la phase gazeuse étant guidée dans l'unité d'absorbants (1) et la phase liquide continuant à être portée à la température nécessaire à la désorption et étant amenée à l'unité de désorption (16) en vue de sa régénération.

2. Procédé selon la revendication 1, **caractérisé en ce que** le liquide lavant chargé et soutiré après l'absorption traverse un premier échangeur de chaleur (9) dans lequel il est porté à la température de séparation nécessaire au moyen du liquide lavant régénéré amené à contre-courant et faisant office d'agent caloporteur, et est introduit pour finir tangentiellement dans le séparateur centrifuge (11).

3. Procédé selon l'une quelconque des revendications 1 ou 2, **caractérisé en ce que** la phase liquide évacuée du séparateur centrifuge (11) traverse un second échangeur de chaleur (14) dans lequel il est porté à la température de désorption nécessaire au moyen du liquide lavant régénéré amené à contre-courant et faisant office d'agent caloporteur, et est introduit pour finir dans l'unité de désorption (16).

4. Procédé selon l'une quelconque des revendications 1 à 3, **caractérisé en ce que** la pression de la phase gazeuse est réglée dans le séparateur centrifuge, le réglage de la pression permettant de compenser les fluctuations de température dans le séparateur centrifuge (11) et les modifications du chargement du liquide lavant amené.

5. Procédé selon l'une quelconque des revendications 1 à 4, **caractérisé en ce qu'**en régime fixe, la pression est réglée dans le séparateur centrifuge (11) de manière à laisser s'échapper une quantité de gaz prédéfinie et à modifier la pression si on souhaite modifier la quantité de gaz d'échappement.

6. Procédé selon l'une quelconque des revendications 2 à 5, **caractérisé en ce que** la température de la solution lavante chargée est surveillée dans le premier échangeur de chaleur (9) et que l'amenée de chaleur est commandée en fonction de la température mesurée.

7. Procédé selon l'une des revendications 1 à 6, **caractérisé en ce qu'**on utilise un cyclone (11) comme séparateur centrifuge.

8. Procédé selon l'une des revendications 1 à 6, **caractérisé en ce qu'**on utilise plusieurs cyclones fonctionnant soit en série soit en parallèle.

9. Procédé selon l'une des revendications 1 à 8, **caractérisé en ce qu'**on utilise un liquide lavant contenant des substances chimiques liant le CO₂ sous forme de carbonate ou d'hydrogénocarbonate.

10. Procédé selon la revendication 9, **caractérisé en ce qu'**on utilise comme substances chimiques, seules ou en mélange, des amines primaires, secondaires ou tertiaires, des sels alcalins d'acides aminés, des solutions de carbonate alcalin.

11. Dispositif pour la mise en oeuvre du procédé selon l'une quelconque des revendications 1 à 10, composé d'une unité d'absorbants comprenant au moins un absorbant (1) et d'une unité de désorbants comprenant au moins un désorbant (16), la section inférieure (2) de l'absorbant (1) étant reliée au désorbant (16) par l'intermédiaire d'une conduite (7) guidant le liquide lavant chargé dans laquelle est implanté au moins un échangeur de chaleur, et une conduite (6) guidant le liquide lavant épuré menant du désorbant (16) à la tête de l'absorbant (1), **caractérisé en ce qu'**au moins un séparateur centrifuge (11) est implanté dans la conduite (7) guidant le liquide lavant chargé après le premier échangeur de chaleur (9), la conduite d'amenée (10) débouchant tangentiellement dans le séparateur centrifuge (11), une conduite de gaz d'échappement (12) menant à la section inférieure (2) de l'absorbant (1) est disposée à la tête du séparateur centrifuge (11), et la conduite de liquide (13) sortant du séparateur centrifuge (11) dans laquelle est implanté un second échangeur de chaleur (14) est reliée à la tête du désorbant (16).

12. Dispositif selon la revendication 11, **caractérisé en ce qu'**un seul cyclone (11) ou plusieurs cyclones montés en série ou en parallèle sont disposés sous forme de séparateur centrifuge.

13. Dispositif selon l'une quelconque des revendications 11 ou 12, **caractérisé en ce qu'**une plaque d'amortissement (27) est disposée au centre de la section inférieure du séparateur centrifuge (11) de manière à former, entre la paroi du séparateur centrifuge (11) et le bord extérieur de la plaque d'amortissement (27) un canal annulaire (28) étroit faisant office d'orifice d'échappement pour la phase liquide.

14. Dispositif selon l'une quelconque des revendications 11 à 13, **caractérisée en ce qu'**un tube plongeur (25) relié à une vanne de purge (26) commandée par flotteur et éventuellement à une pompe à vide est disposé dans la section supérieure du séparateur centrifuge (11).

15. Dispositif selon l'une quelconque des revendications 11 à 14, **caractérisé en ce qu'**un débitmètre (19) est disposé dans la conduite de gaz d'échappement (12) du séparateur centrifuge (11) et un capteur de pression (20) ainsi qu'une vanne de régulation (21), reliés l'un à l'autre au niveau de la technique de régulation, sont disposés dans la conduite (13) évacuant le liquide du séparateur centrifuge (11).
